# EUROPEAN PATENT SPECIFICATION

(11) **EP 2 780 477 B1**
(45) Date of publication and mention of the grant of the patent: **14.03.2018**
(21) Application number: 12849418.4
(22) Date of filing: 15.11.2012
(51) Int. Cl.: C12Q 1/68, C12N 15/11, C12Q 1/04

(54) **SELECTIVE DETECTION OF NOROVIRUS**
SELEKTIVE NOROVIRUS-ERKENNUNG
DÉTECTION SÉLECTIVE DE NOROVIRUS

(30) Priority: 15.11.2011 US 201161560077 P
(43) Date of publication of application: 24.09.2014
(73) Proprietor: The Government of the United States of America as Represented by the Secretary of the Department of Health and Human Services, Atlanta, GA (US)
(72) Inventor: VINJE, Jan, Decatur, GA 30033 (US); GREGORICUS, Nicole, Atlanta, GA 30002 (US); CHHABRA, Preeti, Atlanta, GA 30329 (US); BARCLAY, Leslie, Atlanta, GA 30340 (US)
(74) Representative: Høiberg P/S
(86) International application number: PCT/US2012/065269
(87) International publication number: WO 2013/074785

(56) References cited:
- KR-B1- 100 818 275
- US-A1- 2005 048 475
- US-A1- 2006 110 724
- US-A1- 2008 254 443
- US-A1- 2010 304 377
- ROLFE ET AL: "An internally controlled, one-step, real-time RT-PCR assay for norovirus detection and genogrouping", JOURNAL OF CLINICAL VIROLOGY, ELSEVIER, AMSTERDAM, NL, vol. 39, no. 4, 19 July 2007 (2007-07-19), pages 318-321, XP022164813, ISSN: 1386-6532, DOI: 10.1016/J.JCV.2007.05.005
- J. DREIER ET AL: "Enhanced Reverse Transcription-PCR Assay for Detection of Norovirus Genogroup I", JOURNAL OF CLINICAL MICROBIOLOGY, vol. 44, no. 8, 1 August 2006 (2006-08-01) , pages 2714-2720, XP055193191, ISSN: 0095-1137, DOI: 10.1128/JCM.00443-06
- EVERARDO VEGA: "Novel Surveillance Network for Norovirus Gastroenteritis Outbreaks, United States", EMERGING INFECTIOUS DISEASES, 1 August 2011 (2011-08-01), XP055193195, ISSN: 1080-6040, DOI: 10.3201/eid1708.101837
- ANNA CHARLOTTE SCHULTZ ET AL: "Development and evaluation of novel one-step TaqMan realtime RT-PCR assays for the detection and direct genotyping of genogroup I and II noroviruses", JOURNAL OF CLINICAL VIROLOGY, ELSEVIER, AMSTERDAM, NL, vol. 50, no. 3, 1 December 2010 (2010-12-01), pages 230-234, XP028363225, ISSN: 1386-6532, DOI: 10.1016/J.JCV.2010.12.001 [retrieved on 2010-12-07]
- VINCENT R. HILL ET AL: "Detection of GI and GII Noroviruses in Ground Water Using Ultrafiltration and TaqMan Real-time RT-PCR", FOOD AND ENVIRONMENTAL VIROLOGY, vol. 2, no. 4, 21 November 2010 (2010-11-21), pages 218-224, XP055193220, ISSN: 1867-0334, DOI: 10.1007/s12560-010-9049-y
- A. A. TRUJILLO ET AL: "Use of TaqMan Real-Time Reverse Transcription-PCR for Rapid Detection, Quantification, and Typing of Norovirus", JOURNAL OF CLINICAL MICROBIOLOGY, vol. 44, no. 4, 1 April 2006 (2006-04-01), pages 1405-1412, XP055193219, ISSN: 0095-1137, DOI: 10.1128/JCM.44.4.1405-1412.2006
- KAGEYAMA T ET AL: "Broadly reactive and highly sensitive assay for Norwalk-like viruses based on real-time quantitative reverse transcription-PCR", JOURNAL OF CLINICAL MICROBIOLOGY, AMERICAN SOCIETY FOR MICROBIOLOGY, US, vol. 41, no. 4, 1 April 2003 (2003-04-01), pages 1548-1557, XP002326299, ISSN: 0095-1137, DOI: 10.1128/JCM.41.4.1548-1557.2003
- PANG X L ET AL: "Multiplex real time RT-PCR for the detection and quantitation of norovirus genogroups I and II in patients with acute gastroenteritis", JOURNAL OF CLINICAL VIROLOGY, ELSEVIER, AMSTERDAM, NL, vol. 33, no. 2, 1 June 2005 (2005-06-01), pages 168-171, XP027704004, ISSN: 1386-6532 [retrieved on 2005-06-01]
- STALS A ET AL: "Multiplex real-time RT-PCR for simultaneous detection of GI/GII noroviruses and murine norovirus 1", JOURNAL OF VIROLOGICAL METHODS, ELSEVIER BV, NL, vol. 161, no. 2, 1 November 2009 (2009-11-01), pages 247-253, XP026501538, ISSN: 0166-0934, DOI: 10.1016/J.JVIROMET.2009.06.019 [retrieved on 2009-06-27]
- PUSCH D ET AL: "Detection of enteric viruses and bacterial indicators in German environmental waters", ARCHIVES OF VIROLOGY ; OFFICIAL JOURNAL OF THE VIROLOGY DIVISIONOF THE INTERNATIONAL UNION OF MICROBIOLOGICAL SOCIETIES, SPRINGER-VERLAG, VI, vol. 150, no. 5, 1 May 2005 (2005-05-01), pages 929-947, XP019378515, ISSN: 1432-8798, DOI: 10.1007/S00705-004-0467-8
- HOEHNE M ET AL: "DETECTION AND CHARACTERIZATION OF NOROVIRUS OUTBREAKS IN GERMANY: APPLICATION OF A ONE-TUBE RT-PCR USING A FLUOROGENIC REAL-TIME DETECTION SYSTEM", JOURNAL OF MEDICAL VIROLOGY, JOHN WILEY & SONS, INC, US, vol. 72, no. 2, 1 January 2004 (2004-01-01), pages 312-319, XP002326300, ISSN: 0146-6615, DOI: 10.1002/JMV.10573
- JENS DREIER ET AL: "Use of bacteriophage MS2 as an internal control in viral reverse transcription-PCR assays", JOURNAL OF CLINICAL MICROBIOLOGY, AMERICAN SOCIETY FOR MICROBIOLOGY, US, vol. 43, no. 9, 1 September 2005 (2005-09-01), pages 4551-4557, XP002639742, ISSN: 0095-1137

## Description

### TECHNICAL FIELD

This disclosure relates generally to processes for detection of foreign organisms in biological or environmental samples. More specifically, the disclosure relates to selective detection of norovirus. Processes are described for rapid and sensitive detection of norovirus in biological samples and quantification thereof. Diagnostic kits are provided for detection of norovirus in a clinical, laboratory, or field setting.

BACKGROUND Noroviruses are the primary cause of epidemic viral gastroenteritis and the leading cause of foodborne outbreaks in the United States (1-3). Although the course of disease is in most cases self-limiting, young, elderly, and immunocompromised persons are at risk for complications caused by severe vomiting and diarrhea (4-8). In addition to the clinical impact of norovirus disease, the economic effects in lost wages, time, and intervention procedures (e.g., clean-up costs and recalls) can be significant (9-11). Although norovirus outbreaks occur year-round, they are more common during the winter months (12-14).

Noroviruses are genetically classified into 5 genogroups, GI-GV, with genogroup I (GI) and genogroup II (GII) strains responsible for most human disease (2,15). GII viruses can be further divided into at least 19 genotypes, of which GII.4 is responsible for >85% of outbreaks (14,16), although other genotypes and viruses continue to circulate and cause sporadic disease in children (17-19). Over the past 15 years, new GII.4 variants have been identified; several have been associated with a global increase in the number of outbreaks (15). The last pandemic GII.4 variant, GII.4 2006b or GII.4 Minerva, was identified in late 2005/early 2006 and has been the predominant outbreak strain in the United States since then. The successive displacement of GII.4 variants suggests that population immunity is driving the evolution of GII.4 viruses (20,21), and the emergence of a new variant will cause an increase in the number of outbreaks in an immunologically naive population.

It is not fully understood why some GII.4 variants become pandemic whereas others do not. The combination of novel antigenic sites in protruding regions of the capsid (centered around amino acids 295 and 396) and the change or expansion of a susceptible population may be responsible for the emergence of pandemic variants (20,22). The latter theory has been supported by the discovery that different norovirus strains may have different histo-blood group antigen (HBGA) binding patterns and that nonsecretors are not susceptible to infection with certain genotypes or variants (23). Most mutations between genotypes and variants occur in the P2 region of the major capsid viral protein (VP), VP1, which contains the HBGA binding sites.

Since 2008, all 50 states have had the laboratory capacity for norovirus testing while the Centers for Disease Control and Prevention (CDC) National Calicivirus Laboratory (NCL) provides laboratory support to states that do not have in-house capacity for norovirus strain typing. Recent studies on the molecular epidemiology of norovirus in the US have been based on specimens from a subset of outbreaks that were submitted to CDC (13,24,25). To enhance and harmonize norovirus outbreak surveillance, CDC and its state partners have developed a national norovirus outbreak surveillance network, CaliciNet. CaliciNet was developed to improve standardized typing of norovirus outbreaks, assist in linking geographically different clusters of norovirus illness, allow rapid classification and identification of new norovirus strains, and establish a comprehensive strain surveillance network in the United States.

While the prior assays for detecting and identifying noroviruses in biological samples have some level of effectiveness, these assays suffer from less than optimal detection capabilities. Without being bound to any one particular theory, co-extraction of RT-PCR inhibitors during viral RNA extraction may complicate positive identification of norovirus in a sample, and lead to false negative results. In addition, the fluorescence of the GI component of the prior GI-GII duplex assays is significantly lower than the GII fluorescence and may lead to false negative data interpretation by the performing technician. As such, there is a need for new materials and methods for the detection and identification of norovirus in a sample.

### SUMMARY

The present invention is as defined in the claims. The following summary is provided to facilitate an understanding of some of the innovative features unique to the present disclosure and is not intended to be a full description. A full appreciation of the various aspects of the disclosure can be gained by taking the entire specification, claims, drawings, and abstract as a whole.

The above deficiencies of prior assays for the detection of a norovirus in a sample are effectively addressed by the compositions and processes described and claimed herein. The compositions and processes provided result in robust detection of a norovirus of GI, GII, or both, when present in a sample such as a biological sample derived or obtained from a subject that may or may not be infected with a norovirus. The process and compositions may be used either *in vitro* or *in vivo.* In some embodiments of the present disclosure, compositions and processes are used exclusively *in vitro.* Processes of diagnosis are optionally performed *in vitro.*

An *in vitro* process of detecting norovirus in a sample is provided including producing an amplification product by amplifying a norovirus nucleotide sequence using a forward primer that hybridizes to a norovirus nucleotide sequence, and a reverse primer that hybridizes to a region within said norovirus nucleotide sequence, under conditions suitable for a polymerase chain reaction; and detecting said amplification product to detect the norovirus in the sample using a first probe of SEQ ID NO: 3. In some embodiments of the present disclosure two probes are used with a first probe of SEQ ID NO: 3 and a second probe of SEQ ID NO: 12.

A forward primer is optionally the sequence of SEQ ID NO: 1, SEQ ID NO: 4, SEQ ID NO: 10, or any combination the primers, optionally with the proviso that at least one of SEQ ID NO: 1 or SEQ ID NO: 4 are used. A reverse primer optionally has the sequence of SEQ ID NO: 2, SEQ ID NO: 5, SEQ ID NO: 11, or a combination of the primers. The step of detecting is by using a probe of SEQ ID NO: 6, SEQ ID NO: 3, SEQ ID NO: 12, or any combinations these probe sequences with the proviso that at least SEQ ID NO: 2 or 12 is used.

The processes are optionally used to detect the presence or absence of a norovirus in a sample, optionally to diagnose norovirus infection or the absence thereof in a subject, or combinations thereof. A sample is optionally obtained from a subject or is not obtained from a subject but is or is not supplemented with a norovirus prior to use in a process.

In some embodiments of the present disclosure, a second amplification product is produced optionally by the same process as a first amplification product. A second amplification product may be the result of the presence of a second or additional norovirus genotype in the sample or the subject.

Processes of detecting a first or second amplification product, or both are optionally performed in the presence of a quality control organism that is added to the sample, optionally before detection. Such embodiments of the present disclosure include adding a quantity of control organism to the sample, producing a control amplification product by amplifying a control nucleotide sequence using a control forward primer that hybridizes to a control nucleotide sequence, and a control reverse primer that hybridizes to a region within the control nucleotide sequence, under conditions suitable for a polymerase chain reaction; and detecting the control amplification product to detect the control organism in the sample. The quality control organism is optionally an RNA virus. In some embodiments of the present disclosure, the quality control organism is RNA coliphage MS2. Optionally, the control amplification product is generated by PCR amplification of a purified norovirus, or portion thereof.

Any of these processes are optionally used to detect the presence or absence of a norovirus of GI or GII, or both.

In some embodiments of the present disclosure, the step of detecting is by gel electrophoresis, Southern blotting, liquid chromatography, mass spectrometry, liquid chromatography/mass spectrometry, static fluorescence, dynamic fluorescence, high performance liquid chromatography, ultra-high performance liquid chromatography, enzyme-linked immunoadsorbent assay or other immunoassay, real-time PCR, nucleotide sequencing, or combinations thereof.

Isolated nucleotides are also provided that may be provided alone or included in a kit, optionally for detection of a norovirus in a sample, diagnosis of norovirus infection in a subject, preparation of a composition for diagnosis of a norovirus infection in a subject, or combinations thereof. An isolated nucleotide optionally has a sequence of SEQ ID NO: 3. Optionally, a kit includes any combination of isolated nucleotides with SEQ ID NO: 1, 2, 3, 4, 5, 7, 8, 10, or 11. In some embodiments of the present disclosure, an isolated nucleotide sequence optionally excludes additional or substitute nucleic acids.

### BRIEF DESCRIPTION OF THE DRAWINGS

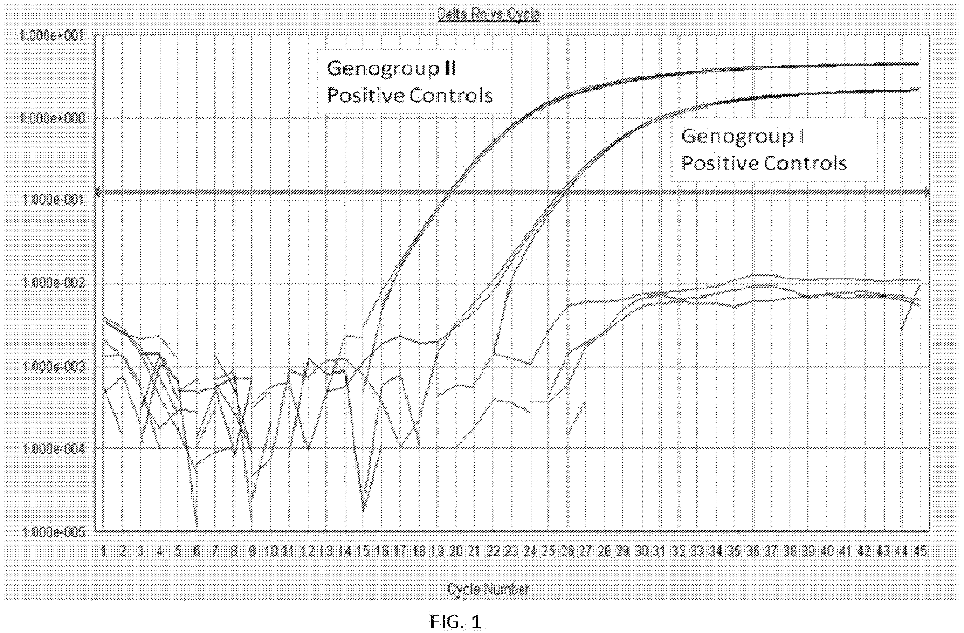

FIG. 1 illustrates amplification of GI and GII noroviruses using a process and compositions according to one embodiment of the disclosure.

### DETAILED DESCRIPTION

The present invention is as defined in the claims. The following description of particular embodiment(s) of the present disclosure is merely exemplary in nature and is in no way intended to limit the scope of the disclosure, its application, or uses, which may, of course, vary. The disclosure is described with relation to the non-limiting definitions and terminology included herein. These definitions and terminology are not designed to function as a limitation on the scope or practice of the disclosure but are presented for illustrative and descriptive purposes only. While the processes are described as an order of individual steps or using specific materials, it is appreciated that described steps or materials may be interchangeable such that the description of the disclosure includes multiple parts or steps arranged in many ways as is readily appreciated by one of skill in the art.

The disclosure has utility for the detection of norovirus in a sample. As it is necessary to detect small numbers of norovirus in clinical specimens, sensitive techniques such as PCR may provide a reliable diagnostic system that simultaneously allows for genogroup identification.

Compositions and methods are provided for the sensitive detection of norovirus in samples, such as biological or environmental samples, using techniques involving PCR. Oligonucleotide primers are provided that amplify the most conserved region of norovirus genome with high specificity that are subsequently detectable, optionally by sensitive detection systems.

The following definitional terms are used throughout the specification without regard to placement relative to these terms.

As used herein, the term "variant" defines either a naturally occurring genetic mutant of norovirus gene or gene products, or a recombinantly prepared variation of norovirus gene or gene products. The term "variant" may also refer to either a naturally occurring variation of a given peptide or a recombinantly prepared variation of a given peptide or protein in which one or more amino acid residues have been modified by amino acid substitution, addition, or deletion.

As used herein, the term "analog" in the context of a non-proteinaceous analog defines a second organic or inorganic molecule that possesses a similar or identical function as a first organic or inorganic molecule and is structurally similar to the first organic or inorganic molecule.

As used herein, the term "derivative" in the context of a non-proteinaceous derivative defines a second organic or inorganic molecule that is formed based upon the structure of a first organic or inorganic molecule. A derivative of an organic molecule includes, but is not limited to, a molecule modified, e.g., by the addition or deletion of a hydroxyl, methyl, ethyl, carboxyl or amine group. An organic molecule may also be esterified, alkylated and/or phosphorylated. A derivative also defined as a degenerate base mimicking a C/T mix such as that from Glen Research Corporation, Sterling, VA, illustratively LNA-dA or LNA-dT, or other nucleotide modification known in the art or otherwise.

As used herein, the term "mutant" defines the presence of mutations in the nucleotide sequence of an organism as compared to a wild-type organism. A mutant is a variant.

A "purified" nucleic acid molecule is one that is separated from other nucleic acid molecules that are present in the natural source of the nucleic acid molecule and is often substantially free of other cellular material, or culture medium when produced by recombinant techniques, or substantially free of chemical precursors or other chemicals when chemically synthesized. This term is exclusive of a nucleic acid that is a member of a library that has not been purified away from other library clones containing other nucleic acid molecules.

As used herein, the term "hybridizes under stringent conditions" describes conditions for hybridization and washing under which nucleotide sequences having at least 30%, 35%, 40%, 45%, 50%, 55%, 60%, 65%, 70%, 75%, 80%, 85%, 90%, 95%, or more, base pair matches to each other typically remain hybridized to each other. Illustrative hybridization conditions are described in, for example but not limited to, Current Protocols in Molecular Biology, John Wiley & Sons, N.Y. (1989), 6.3.1 6.3.6.; Basic Methods in Molecular Biology, Elsevier Science Publishing Co., Inc., N.Y. (1986), pp.75 78, and 84 87; and Molecular Cloning, Cold Spring Harbor Laboratory, N.Y. (1982), pp.387 389, and are well known to those skilled in the art. A non-limiting example of stringent hybridization conditions is hybridization in 6x sodium chloride/sodium citrate (SSC), 0.5% SDS at about 60°C followed by one or more washes in 2xSSC, 0.5% SDS at room temperature. Another non-limiting example of stringent hybridization conditions is hybridization in 6x SSC at about 45°C followed by one or more washes in 0.2x SSC, 0.1% SDS at 50 to 65 °C. Other stringent hybridization conditions will be evident to one of ordinary skill in the art based on general knowledge in the art as well as this specification.

An "isolated" or "purified" nucleotide or oligonucleotide sequence is substantially free of cellular material or other contaminating proteins or nucleotide sequences from the cell or tissue source from which the nucleotide is derived, or is substantially free of chemical precursors or other chemicals when chemically synthesized. The language "substantially free of cellular material" includes preparations of a nucleotide/oligonucleotide in which the nucleotide/oligonucleotide is separated from cellular components of the cells from which it is isolated or produced. Thus, a nucleotide/oligonucleotide that is substantially free of cellular material includes preparations of the nucleotide having less than about 30%, 20%, 10%, 5%, 2.5%, or 1%, (by dry weight) of contaminating material. When nucleotide/oligonucleotide is produced by chemical synthesis, it is optionally substantially free of chemical precursors or other chemicals, i.e., it is separated from chemical precursors or other chemicals which are involved in the synthesis of the molecule. Accordingly, such preparations of the nucleotide/oligonucleotide have less than about 30%, 20%, 10%, 5% (by dry weight) of chemical precursors or compounds other than the nucleotide/oligonucleotide of interest. In some embodiments of the present disclosure, a nucleotide/oligonucleotide is isolated or purified.

As used herein, the term "sample" is a portion of a larger source. A sample is optionally a solid, gaseous, or fluidic sample. A sample is illustratively an environmental or biological sample. An environmental sample is illustratively, but not limited to, water, sewage, soil, or air. A "biological sample" is as sample obtained from a biological organism, a tissue, cell, cell culture medium, or any medium suitable for mimicking biological conditions. Non-limiting examples include, feces, saliva, gingival secretions, cerebrospinal fluid, gastrointestinal fluid, mucous, urogenital secretions, synovial fluid, blood, serum, plasma, urine, cystic fluid, lymph fluid, ascites, pleural effusion, interstitial fluid, intracellular fluid, ocular fluids, seminal fluid, mammary secretions, vitreal fluid; nasal secretions; and throat or nasal materials. In some embodiments of the present disclosure, target agents are contained in: feces; urine; serum; plasma; or whole blood.

As used herein, the term "medium" refers to any liquid or fluid sample in the presence or absence of a bacterium. A medium is illustratively a solid sample that has been suspended, solubilized, or otherwise combined with fluid to form a fluidic sample. Non-limiting examples include buffered saline solution, cell culture medium, acetonitrile, trifluoroacetic acid, combinations thereof, or any other fluid recognized in the art as suitable for combination with a cell, a virus, or bacteria, or for dilution of a biological sample or amplification product for analysis.

To determine the percent identity of two nucleic acid sequences, the sequences are aligned for optimal comparison purposes (e.g., gaps can be introduced in the sequence of a first amino acid or nucleic acid sequence for optimal alignment with a second amino acid or nucleic acid sequence). The nucleotides at corresponding nucleotide positions are then compared. When a position in the first sequence is occupied by the same nucleotide as the corresponding position in the second sequence, then the molecules are identical at that position. The percent identity between the two sequences is a function of the number of identical positions shared by the sequences (i.e., % identity=number of identical overlapping positions/total number of positions .times.100%). In some embodiments of the present disclosure, the two sequences are the same length.

The determination of percent identity between two sequences can also be accomplished using a mathematical algorithm. A non-limiting example of a mathematical algorithm utilized for the comparison of two sequences is the algorithm of Karlin and Altschul, 1990, PNAS 87:2264 2268, modified as in Karlin and Altschul, 1993, PNAS. 90:5873 5877. Such an algorithm is incorporated into the NBLAST and XBLAST programs of Altschul et al., 1990, J. Mol. Biol. 215:403. BLAST nucleotide searches are performed with the NBLAST nucleotide program parameters set, e.g., for score=100, wordlength=12 to obtain nucleotide sequences homologous to a nucleic acid molecules of the present disclosure. BLAST protein searches are performed with the XBLAST program parameters set, e.g., to score 50, wordlength=3 to obtain amino acid sequences homologous to a protein molecule of the present disclosure. To obtain gapped alignments for comparison purposes, Gapped BLAST are utilized as described in Altschul et al., 1997, Nucleic Acids Res. 25:3389 3402. Alternatively, PSI BLAST is used to perform an iterated search which detects distant relationships between molecules (Id.). When utilizing BLAST, Gapped BLAST, and PSI Blast programs, the default parameters of the respective programs (e.g., of XBLAST and NBLAST) are used (see, e.g., the NCBI website). Another non-limiting example of a mathematical algorithm utilized for the comparison of sequences is the algorithm of Myers and Miller, 1988, CABIOS 4:11 17. Such an algorithm is incorporated in the ALIGN program (version 2.0) which is part of the GCG sequence alignment software package. When utilizing the ALIGN program for comparing amino acid sequences, a PAM120 weight residue table, a gap length penalty of 12, and a gap penalty of 4 is used.

The percent identity between two sequences is determined using techniques similar to those described herein or otherwise known in the art, with or without allowing gaps. In calculating percent identity, typically only exact matches are counted.

As used herein, the terms "subject" and "patient" are synonymous and refer to a human or non-human animal, optionally a mammal including a human, a non-primate such as cows, pigs, horses, goats, sheep, cats, dogs, avian species and rodents; and a non-human primate such as monkeys, chimpanzees, and apes; and a human, also optionally denoted specifically as a "human subject".

Processes are described that provide a rapid, specific, and sensitive assay for detection of norovirus in a sample by amplifying one or more norovirus nucleotide sequences by processes similar to the polymerase chain reaction (PCR). Processes are similarly provided for diagnosing the presence or absence of norovirus infection in a subject. The presence of norovirus detected in a sample from the subject diagnoses or confirms a prior diagnosis of infection of the subject by norovirus. The absence of norovirus in a sample from a subject diagnoses the absence of an infection of the subject by norovirus.

Some embodiments of the present disclosure include the screening for the presence or absence of a control organism in the same or a second sample from the same subject or the same environment. A second sample is optionally obtained and used in a process in parallel, or in sequence with a first sample. A control organism is optionally a bacteria, virus, or other control organism or cell. Some embodiments of the present disclosure use a bacteriophage as a control organism. Optionally, an RNA coliphage is used as a control organism. A process optionally includes assaying a sample for the presence or absence of a control organism. The presence of a control organism optionally indicates the absence of reverse transcriptase or other process inhibitors in the sample. The absence of a control organism optionally indicated the presence of reverse transcriptase or other process inhibitors in the sample.

An oligonucleotide forward primer with a nucleotide sequence complementary to a sequence in a norovirus genetic sequence or cDNA product produced therefrom is hybridized to its complementary sequence and extended. A nucleotide sequence is complementary if it hybridizes under stringent conditions. Similarly, a reverse oligonucleotide primer complementary to a second strand of a sequence in a norovirus genetic sequence or cDNA product produced therefrom is hybridized and extended. This system allows for amplification of specific norovirus nucleotide sequences and is suitable for simultaneous or sequential detection systems.

The present disclosure relates to the use of the sequence information of norovirus for diagnostic, research or other processes, either *in vitro* or *in vivo.* In particular, the present disclosure provides a process for detecting the presence or absence of nucleic acid molecules of norovirus which in some embodiments of the present disclosure include natural or artificial variants, analogs, or derivatives thereof, in a sample. In some embodiments of the present disclosure, processes involve obtaining a biological sample from one or more of various sources and contacting the sample with a compound or an agent capable of detecting a nucleic acid sequence of norovirus, natural or artificial variants, analogs, or derivatives thereof, such that the presence or absence of norovirus, natural or artificial variants, analogs, or derivatives thereof, is detected in the sample. Optionally, infection by norovirus GI of GII is diagnosed by positively detecting one or more norovirus in the sample. In some embodiments of the present disclosure, the presence of norovirus, natural or artificial variants, analogs, or derivatives thereof, is detected in the sample using a PCR reaction or real-time polymerase chain reaction (qPCR), optionally following a reverse transcription (RT) reaction from norovirus RNA to copy DNA, including primers that are constructed based on a conserved region of the norovirus genome. In a non-limiting embodiment of the present disclosure, a forward primer designed to be successful for selective amplification in a PCR based assay such as in a PCR process is illustratively 5'-CGYTGGATGCGITTYCATGA-3' (SEQ ID NO: 1), 5'-CARGARBCNATGTTYAGRTGGATGAG-3' (SEQ ID NO: 4), 5'-CCATGTTCCGTTGGATGC-3' (SEQ ID NO: 10), or combinations thereof. A nucleotide denoted as: R represents either A or G; B represents C, G or T; N represents A, G, C or T; Y represents C, T, or U, and I represents inosine or deoxyinosine. In some embodiments of the present disclosure, a reverse primer designed to be successful for selective amplification in a PCR based assay such as in a PCR process is illustratively 5'-CTTAGACGCCATCATCATTYAC-3' (SEQ ID NO: 2), 5'-TCGACGCCATCTTCATTCACA-3' (SEQ ID NO: 5), 5'-TCCTTAGACGCCATCATCAT-3' (SEQ ID NO: 11), or combinations thereof. In some embodiments of the present disclosure, the primer pairs used in a process are the nucleic acid sequences of SEQ ID NOs: 1 and 2, 4 and 5, 10 and 11, or combinations thereof. It is appreciated that SEQ ID NOs: 1 and 10 are optionally substitutable or supplementary in a process. It is appreciated that SEQ ID NOs: 2 and 11 are optionally substitutable or supplementary in a process. As used herein, the term "amplify" is defined as producing one or more copies of a target molecule, or a complement thereof. A nucleic acid such as DNA or RNA is amplified to produce one or more amplification products. Illustratively, a forward primer and an optional reverse primer are contacted with a target under conditions suitable for a polymerase chain reaction to produce an amplification product.

An agent for detecting norovirus nucleic acid sequences is a labeled nucleic acid probe capable of hybridizing to a portion of the norovirus ORF, or amplification products derived therefrom. In some embodiments of the present disclosure, the nucleic acid probe is a nucleic acid molecule of the nucleic acid sequence of 5'-TGGACAGGRGAYCGC-3' (SEQ ID NO: 3), which sufficiently specifically hybridizes under stringent conditions to norovirus nucleic acid sequence amplified from GI norovirus. In some embodiments of the present disclosure, the nucleic acid probe is a nucleic acid molecule of the nucleic acid sequence of 5'-TGGGAGGGCGATCGCAATCT-3' (SEQ ID NO: 6), which sufficiently specifically hybridizes under stringent conditions to norovirus nucleic acid sequence amplified from GII norovirus. In some embodiments of the present disclosure, the nucleic acid probe is a nucleic acid molecule of the nucleic acid sequence of 5'-GGACAGGAGAYCGCRATCT-3' (SEQ ID NO: 12), which sufficiently specifically hybridizes under stringent conditions to norovirus nucleic acid sequence amplified from GI noroviruses. A probe is optionally labeled with a fluorescent molecule such as a fluorescein illustratively 6-carboxyfluorescein (FAM), an indocarbocyanine illustratively that sold under the tradename QUASAR-670 (QUA), a hexaflurocine such as 6-carboxyhexafluorescein (HEX), or other fluorophore molecule and optionally a quencher. A quencher is appreciated to be matched to a fluorophore. Illustrative examples of a quencher in clued the black hole quenchers BHQ1, and BHQ2, or the dihydrocyclopyrroloindole tripeptide minor groove binder (MGB). Other fluorophores and quenchers are known in the art and are similarly operable herein. In some embodiments of the present disclosure, a fluorophore for SEQ ID NOs: 3 or 12 is limited to 6-carboxyfluorescein and a quencher is limited to MGB. In some embodiments of the present disclosure, a fluorophore for SEQ ID NO: 6 is limited to QUA, and a quencher is BHQ2. Primers and probes are further illustrated in Table 1.

**Table 1:**

| **Genogroup** | **Nucleotide** | **Function** | **Sequence** | **SEQ ID NO:** |
|---|---|---|---|---|
| GI | Cog1F | Primer | 5'-CGYTGGATGCGITTYCATGA-3' | 1 |
| | Cog1R | Primer | 5'-CTTAGACGCCATCATCATTYAC-3' | 2 |
| | Cog1NF | Primer | 5'-CCATGTTCCGTTGGATGC-3' | 10 |
| | CoglNR | Primer | 5'-TCCTTAGACGCCATCATCAT-3' | 11 |
| | Ring 1E | Probe | 5'-TGGACAGGRGAYCGC-3' | 3 |
| | NV1LCpr | Probe | 5'-GGACAGGAGAYCGCRATCT-3 | 12 |
| GII | Cog2F | Primer | 5'-CARGARBCNATGTTYAG RTGGATGAG-3' | 4 |
| | Cog2R | Primer | 5'-TCGACGCCATCTTCATTCACA-3' | 5 |
| | Ring 2 | Probe | 5'-TGGGAGGGCGATCGCAATCT-3' | 6 |

Primers are optionally used for the sequencing of a norovirus. Illustratively, primers for PCR include a forward primer of SEQ ID NO: 1, 4, 10 or combinations thereof, and a reverse primer of SEQ ID NO: 2, 5, 11, or combinations thereof.

Processes optionally involve a RT real time-PCR assay (RT-qPCR), which is a quantitative assay. In some embodiments of the present disclosure, the PCR assay is a TaqMan assay (Holland et al., PNAS 88(16):7276 (1991)). It is appreciated that the processes are amenable to performance on other RT-qPCR systems and protocols that use alternative reagents illustratively including, but not limited to Molecular Beacons probes, Scorpion probes, multiple reporters for multiplex PCR, combinations thereof, or other DNA or RNA detection systems.

The assays are performed on an instrument designed to perform such assays, for example those available from Applied Biosystems (Foster City, Calif.). In some embodiments of the present disclosure, a RT-qPCR assay is used to detect the presence of norovirus, natural or artificial variants, analogs, or derivatives thereof, in a sample by subjecting the norovirus nucleic acid from the sample to PCR reactions using specific primers, and detecting the amplified product using a probe. In some embodiments of the present disclosure, the probe is a TaqMan probe which consists of an oligonucleotide with a 5'-reporter dye and a 3'-quencher dye.

A fluorescent reporter dye, such as FAM dye (illustratively 6-carboxyfluorescein), is covalently linked, optionally to the 5' end of the oligonucleotide probe. Other dyes illustratively include TAMRA, AlexaFluor dyes such as AlexaFluor 495 or 590, Cascade Blue, Marina Blue, Pacific Blue, Oregon Green, Rhodamine, Fluoroscein, TET, HEX, Cy5, Cy3, and Tetramethylrhodamine. A reporter is optionally quenched by a dye at the 3' end or other non-fluorescent quencher. Quenching molecules are optionally suitably matched to the fluorescence maximum of the dye. Any suitable fluorescent probe for use in RT-qPCR detection systems is illustratively used in some embodiments of the instant disclosure. Similarly, any quenching molecule for use in RT-qPCR systems is illustratively operable in some embodiments of the present disclosure. In some embodiments of the present disclosure, a 6-carboxyfluorescein reporter dye is present at the 5'-end and matched to BLACK HOLE QUENCHER (BHQ1, Biosearch Technologies, Inc., Novato, CA.) The fluorescence signals from these reactions are captured at the end of extension steps as PCR product is generated over a range of the thermal cycles, thereby allowing the quantitative determination of the bacterial load in the sample based on an amplification plot.

The norovirus nucleic acid sequences are optionally reverse transcribed and amplified before or simultaneous with being detected. The term "amplified" defines the process of making multiple copies of the nucleic acid from a single or lower copy number of nucleic acid sequence molecule. The amplification of nucleic acid sequences is carried out *in vitro* by biochemical processes known to those of skill in the art, illustratively by PCR techniques. The amplification agent may be any compound or system that will function to accomplish the synthesis of primer extension products, including enzymes. Suitable enzymes for this purpose include, for example, *E. coli* DNA polymerase I, Taq polymerase, Klenow fragment of *E*. *coli* DNA polymerase I, T4 DNA polymerase, AmpliTaq Gold DNA Polymerase from Applied Biosystems, other available DNA polymerases, reverse transcriptase (optionally iScript RNase H+ reverse transcriptase, or and an MMLV Reverse Transcriptase such as that sold under the tradename ARRAY SCRIPT from Applied Biosystems, Foster City, CA), ligase, and other enzymes, including heat-stable enzymes (i.e., those enzymes that perform primer extension after being subjected to temperatures sufficiently elevated to cause denaturation). In some embodiments of the present disclosure, the enzyme is hot-start AMPLITAQ GOLD DNA polymerase from Applied Biosystems, Foster City, CA. Suitable enzymes will facilitate combination of the nucleotides in the proper manner to form the primer extension products that are complementary to each mutant nucleotide strand. Generally, the synthesis is initiated at the 3'-end of each primer and proceed in the 5'-direction along the template strand, until synthesis terminates, producing molecules of different lengths. There may be amplification agents, however, that initiate synthesis at the 5'-end and proceed in the other direction, using the same or similar processes. In any event, the processes of the disclosure are not to be limited to the embodiments of amplification described herein.

One process of *in vitro* amplification, which optionally is used according to this disclosure, is the polymerase chain reaction (PCR) described in U.S. Pat. Nos. 4,683,202 and 4,683,195. The term "polymerase chain reaction" refers to a process for amplifying a DNA base sequence using a heat-stable DNA polymerase and two oligonucleotide primers, one complementary to the (+)-strand at one end of the sequence to be amplified and the other complementary to the (-)-strand at the other end. Because the newly synthesized DNA strands can subsequently serve as additional templates for the same primer sequences, successive rounds of primer annealing, strand elongation, and dissociation produce rapid and highly specific amplification of the desired sequence. Many polymerase chain processes are known to those of skill in the art and may be used in the process of the disclosure. For example, RNA is subjected to cycling conditions optionally including reverse transcription for 10 min at 45°C and denaturation for 10 min at 95°C, followed by 45 cycles of 15 s at 95°C and 1 min at 60°C.

The primers for use in amplifying the RNA (or DNA produced therefrom) of norovirus may be prepared using any suitable process, such as conventional phosphotriester and phosphodiester processes or automated embodiments thereof so long as the primers are capable of hybridizing to the nucleic acid sequences of interest. One process for synthesizing oligonucleotides on a modified solid support is described in U.S. Pat. No. 4,458,066. The exact length of primer will depend on many factors, including temperature, buffer, and nucleotide composition. The primer must prime the synthesis of extension products in the presence of the inducing agent for amplification.

Primers used according to the process of the disclosure are complementary to each strand of nucleotide sequence to be amplified. The term "complementary" means that the primers hybridize with their respective strands under conditions that allow the agent for polymerization to function, such as stringent hybridization conditions. In other words, the primers that are complementary to the flanking sequences hybridize with the flanking sequences and permit amplification of the nucleotide sequence. Optionally, the 3' terminus of the primer that is extended is perfectly (100%) base paired with the complementary flanking strand. Probes optionally possess nucleotide sequences complementary to one or more strands of the amplification product of norovirus. Optionally, primers contain the nucleotide sequences of SEQ ID NOs: 1 and 2, 4 and 5, 10 and 11, or combinations thereof. It is appreciated that the complements of SEQ ID NOs: 1 and 2, 4 and 5, 10 and 11, are similarly suitable for use in the instant disclosures. It is further appreciated that oligonucleotide sequences that hybridize with SEQ ID NOs 1, 2, 4, 5, 10 or 11 are also similarly suitable.

Those of ordinary skill in the art will know of various amplification processes that can also be utilized to increase the copy number of target norovirus nucleic acid sequence. The nucleic acid sequences detected in the process of the disclosure are optionally further evaluated, detected, cloned, sequenced, and the like, either in solution or after binding to a solid support, by any process usually applied to the detection of a specific nucleic acid sequence such as another polymerase chain reaction, oligomer restriction (Saiki et al., BioTechnology 3:1008 1012 (1985)), allele-specific oligonucleotide (ASO) probe analysis (Conner et al., PNAS 80: 278 (1983)), oligonucleotide ligation assays (OLAs) (Landegren et al., Science 241:1077 (1988)), RNase Protection Assay, among others. Molecular techniques for DNA analysis have been reviewed (Landegren et al, Science 242:229 237 (1988)). Following DNA amplification, the reaction product may be detected by Southern blot analysis, with or without using radioactive probes. In such a process, for example, a small sample of DNA containing the nucleic acid sequence obtained from the tissue or subject is amplified, and analyzed via a Southern blotting technique. The use of non-radioactive probes or labels is facilitated by the high level of the amplified signal. In some embodiments of the disclosure, one nucleoside triphosphate is radioactively labeled, thereby allowing direct visualization of the amplification product by autoradiography. In some embodiments of the present disclosure, amplification primers are fluorescently labeled and run through an electrophoresis system. Visualization of amplified products is by light detection followed by computer assisted graphic display, without a radioactive signal.

Other methods of detection amplified oligonucleotide illustratively include gel electrophoresis, mass spectrometry, liquid chromatography, fluorescence, luminescence, gel mobility shift assay, fluorescence resonance energy transfer, nucleotide sequencing, enzyme-linked immunoadsorbent assay, affinity chromatography, other chromatography methods, immunoenzymatic methods (Ortiz, A and Ritter, E, Nucleic Acids Res., 1996; 24:3280-3281), streptavidin-conjugated enzymes, DNA branch migration (Lishanski, A, et al., Nucleic Acids Res., 2000; 28(9):e42), enzyme digestion (U.S. Patent No. 5,580,730), colorimetric methods (Lee, K., Biotechnology Letters, 2003; 25:1739-1742), or combinations thereof. A detection signal is produced that is related to the detection method employed, be it RT-qPCR or other detection method. A test sample optionally produces a first detection signal upon amplification of a target. A control sample optionally produces a second detection signal upon amplification of a control molecule.

The term "labeled" with regard to the probe is intended to encompass direct labeling of the probe by coupling (i.e., physically linking) a detectable substance to the probe, as well as indirect labeling of the probe by reactivity with another reagent that is directly labeled. Examples of indirect labeling include detection of a probe using a fluorescently labeled antibody and end-labeling or centrally labeling of a DNA probe with biotin such that it can be detected with fluorescently labeled streptavidin. The detection methods can be used to detect RNA ,genomic nucleic acid, or amplification products thereof, in a sample *in vitro* as well as *in vivo.* For example, *in vitro* techniques for detection of nucleic acid include northern hybridizations, in situ hybridizations, reverse transcription-PCR, real-time-PCR, and DNase protection. *In vivo* techniques for detection of norovirus include introducing into a subject organism a labeled antibody directed against a polypeptide component or directed against a particular nucleic acid sequence of norovirus. For example, the antibody can be labeled with a radioactive marker whose presence and location in the subject organism can be detected by standard imaging techniques, including autoradiography.

The size of the primers used to amplify a portion of the nucleic acid sequence of norovirus or control is at least 5, and often 10, 15, 20, 25, 30 or more nucleotides in length, optionally any value or range between 5 and 30 nucleotides in length. Optionally, the GC ratio is above 30%, 35%, 40%, 45%, 50%, 55%, or 60% so as to prevent hair-pin structure on the primer. The amplicon is optionally of sufficient length to be detected by standard molecular biology methodologies. The forward primer is optionally shorter than the reverse primer or vice versa. Techniques for modifying the Tₘ of either primer are operable herein. An illustrative forward primer contains LNA-dA and LNA-dT (Glen Research Corporation) so as to match Tₘ with a corresponding alternate primer.

An inventive process uses a polymerization reaction which employs a nucleic acid polymerizing enzyme, illustratively a DNA polymerase, RNA polymerase, reverse transcriptase, or mixtures thereof. It is further appreciated that accessory proteins or molecules are present to form the replication machinery. A polymerizing enzyme is optionally a thermostable polymerase or thermodegradable polymerase. Use of thermostable polymerases is well known in the art such as Taq polymerase available from Invitrogen Corporation, Carlsbad, CA. Thermostable polymerases allow a polymerization reaction to be initiated or shut down by changing the temperature other condition in the reaction mixture without destroying activity of the polymerase.

Accuracy of the base pairing of DNA sequence amplification is provided by the specificity of the enzyme. Error rates for Taq polymerase tend to be false base incorporation of 10⁻⁵ or less. (Johnson, Annual Reviews of Biochemistry, 1993: 62:685-713; Kunkel, Journal of Biological Chemistry, 1992; 267:18251-18254). Specific examples of thermostable polymerases illustratively include those isolated from *Thermus aquaticus, Thermus thermophilus, Pyrococcus woesei, Pyrococcus furiosus, Thermococcus litoralis* and *Thermotoga maritima.* Thermodegradable polymerases illustratively include *E. coli* DNA polymerase, the Klenow fragment of *E. coli* DNA polymerase, T4 DNA polymerase, T7 DNA polymerase and other examples known in the art. It is recognized in the art that other polymerizing enzymes are similarly suitable illustratively including *E. coli,* T7, T3, SP6 RNA polymerases and AMV, M-MLV, and HIV reverse transcriptases.

The polymerases are optionally bound to the primer. When the norovirus sequence is a single-stranded DNA molecule due to heat denaturing, the polymerase is bound at the primed end of the single-stranded nucleic acid at an origin of replication. A binding site for a suitable polymerase is optionally created by an accessory protein or by any primed single-stranded nucleic acid.

In some embodiments of the present disclosure, detection of PCR products is achieved by mass spectrometry. Mass spectrometry has several advantages over real-time PCR systems in that it can be used to simultaneously detect the presence of norovirus and decipher mutations in target nucleic acid sequences allowing identification and monitoring of emerging strains. Further, mass spectrometers are prevalent in the clinical laboratory. Similar to fluorescence based detection systems, mass spectrometry is capable of simultaneously detecting multiple amplification products for a multiplexed and controlled approach to accurately quantifying components of biological or environmental samples.

Multiple mass spectrometry platforms are suitable for use in the disclosure illustratively including matrix assisted laser desorption ionization time of flight mass spectrometry (MALDI), electrospray mass spectrometry, electrospray ionization-Fourier transform ion cyclotron resonance mass spectrometry (ESI-FTICR), multi-stage mass spectrometry fragmentation analysis (MS/MS), mass spectrometry coupled with liquid chromatography such as high performance liquid chromatography mass spectrometry (HPLC) and ultra performance liquid chromatography isotope dilution tandem mass spectrometry (UPLC-ID/MS/MS), and variations thereof.

It is appreciated that numerous other detection processes are similarly suitable for measuring an amplification product by detecting a detection signal. Illustrative examples include, but are not limited to, liquid chromatography, mass spectrometry, liquid chromatography/mass spectrometry, static fluorescence, dynamic fluorescence, high performance liquid chromatography, ultra-high performance liquid chromatography, enzyme-linked immunoadsorbent assay, real-time PCR (qPCR), gel electrophoresis, or combinations thereof.

Optionally, PCR amplification products are generated using complementary forward and reverse oligonucleotide primers. In a non-limiting example, norovirus GI genetic sequences or fragments thereof are amplified by the primer pair SEQ ID NOs: 1 and 2, 10 and 11, or combinations thereof. In a non-limiting example, norovirus GII genetic sequences or fragments thereof are amplified by the primer pair SEQ ID NOs: 4 and 5. The resulting amplification product(s) is either directly detected such as by a probe, or is subsequently processed and prepared for detection by processes known in the art. It is appreciated that the complements of SEQ ID NOs: 1, 2, 4, 5, 10 or 11 are similarly suitable for use in the disclosure. It is further appreciated that oligonucleotide sequences that hybridize with SEQ ID NOs: 1, 2, 4, 5, 10 or 11 are also similarly suitable.

Optionally, multiple amplification products are simultaneously produced in a PCR reaction that are then available for simultaneous detection and quantification. Thus, multiple detection signals are inherently produced or emitted that are separately and uniquely detected in one or more detection systems. It is appreciated that multiple detection signals are optionally produced in parallel. Optionally, a single biological sample is subjected to analysis for the simultaneous or sequential detection of norovirus genetic sequences. It is appreciated that three or more independent or overlapping sequences are simultaneously or sequentially measured in the inventive processes. Oligonucleotide matched primers (illustratively SEQ ID NOs: 1 and 2) are simultaneously or sequentially added and the biological sample, or a portion thereof, is subjected to proper thermocycling reaction parameters. For detection by mass spectrometry, a single sample of the amplification products from each gene are simultaneously analyzed allowing for rapid and accurate determination of the presence or absence of norovirus. Optionally, analysis by RT-qPCR is employed capitalizing on multiple probes with unique fluorescent signatures. Thus, each gene is detected without interference by other amplification products. This multi-target approach increases confidence in quantification and provides for additional internal control.

In some embodiments of the present disclosure, the processes further involve optionally adding a control organism or portion thereof to a test sample, or to a sample similar to a test sample, and contacting the control organism or portion thereof with a compound or agent capable of detecting the presence of control organism nucleic acid in the sample, and comparing the presence or absence of RNA (or DNA) from the control organism with the presence of RNA in the test sample. Illustratively, a control organism is the RNA coliphage MS2 which is similar in size and morphology (i.e. non-enveloped positive-sense single-stranded RNA virus) as norovirus. MS2 is optionally added to a test sample (e.g. human stool) prior to extraction of RNA from the test sample. The detection of MS2 is optionally performed in parallel with detection of the presence or absence of a norovirus. A control organism is optionally a purified, isolated, or otherwise processed nucleic acid sequence of known concentration optionally including at least a portion of the norovirus sequence, MS2 RNA sequence, or complement thereof, where the nucleic acid sequence or portion thereof will hybridize under stringent conditions with a forward primer, a reverse primer, and, optionally, a probe. A forward primer for MS2 is optionally 5'-TGGCACTACCCCTCTCCGTATTCACG-3' (SEQ ID NO: 7). A reverse primer for MS2 is optionally 5'-GTACGGGCGACCCCACGATGAC-3' (SEQ ID NO: 8). A probe specific for MS2 is optionally 5'-CACATCGATAGATCAAGGTGCCTACAAGC-3' (SEQ ID NO: 9). It is appreciated that complements or nucleotide sequences that hybridize with any of SEQ ID NOs: 7, 8, and 9 are similarly operable and optionally used. It is further appreciated that descriptions of synthesis, modification, substitution, labels, etc. otherwise described herein are applicable to SEQ ID NOs: 7, 8, and 9 as is understood in the art. A control organism is used to produce a control organism amplification product produced either simultaneously with, or sequentially to the norovirus amplification product produced from a target norovirus. The control organism amplification product is optionally detected by a second detection signal by the same or a different method than that used to detect the norovirus amplification product, which is optionally detected by two norovirus genogroup-specific detection signals. Illustratively, a control organism amplification product is detected using a probe that will sufficiently hybridize to an amplification product from a control organism. A control organism probe optionally has one or more labels that are the same or different than that of a norovirus probe, when present. Illustratively, a control organism or portion thereof is subjected to the identical amplification conditions in the same or other parallel analysis, such as on the same instrument, as the norovirus test sample. If the test sample and a sample including a control organism are processed in different reaction chambers, the same probes with the same labels may be used. Sequences of primers and probes for a control reaction in some embodiments of the present disclosure are illustrated in Table 2.

**Table 2:**

| **Strain** | **Nucleotide** | **Function** | **Sequence** | **SEQ ID NO:** |
|---|---|---|---|---|
| coliphage MS2 | MS2.F | primer | 5'-TGGCACTACCCCTCTCCGTATTCACG-3' | 7 |
| | MS2.R | primer | 5'-GTACGGGCGACCCCACGATGAC-3' | 8 |
| | MS2.P | probe | 5'-CACATCGATAGATCAAGGTGCCTACAAGC-3' | 9 |

In some embodiments of the present disclosure, the processes further involve optionally obtaining a control sample from a control subject, contacting a control sample, optionally from said subject, with a compound or agent capable of detecting the presence of norovirus nucleic acid in the sample, and comparing the presence or absence of RNA (or DNA) in the control sample with the presence of RNA in the test sample. A control sample is optionally a portion of a test sample processed in parallel with the test sample. A control sample is optionally a purified, isolated, or otherwise processed nucleic acid sequence of known concentration optionally including at least a portion of the norovirus sequence or complement thereof, where the nucleic acid sequence or portion thereof will hybridize under stringent conditions with a forward primer, a reverse primer, and, optionally, a probe. A control sample is used to produce a complementary amplification product produced either simultaneously with, or sequentially to the first amplification product produced from a target. The complementary amplification product is optionally detected by detecting a second detection signal by the same of a different method than that used to detect the first amplification product. Illustratively, a second amplification product is detected using a second probe of the same or of a different sequence than that use to detect the first amplification product. A second probe optionally has one or more labels that are the same or different than that of a first probe, when present. Illustratively, a control sample is subjected to the identical amplification conditions in the same or other parallel analysis, such as on the same instrument, as the test sample. If the test sample and the control sample are processed in different reaction chambers, the same probes with the same labels may be used.

Some embodiments of the present disclosure include using a nucleic acid calibrator to produce a signal from a known quantity of one or more norovirus nucleic acid molecules. A nucleic acid calibrator is optionally identical to or different from a target molecule. Amplification of a nucleic acid calibrator optionally produces a third (or other) detection signal, the presence of, intensity of, or size of is optionally compared to a norovirus detection signal to quantify the amount of target, or amplification product in the test sample. Optionally, a plurality of nucleic acid calibrators are used. A plurality of nucleic acid calibrators are optionally of differing concentrations such as those suitable to produce a standard curve. The detection signal from the test sample is optionally compared to the standard curve to quantify the amount of amplification product or target in the test sample. A nucleic acid calibrator optionally includes a known amount of norovirus nucleic acid sequence, or a portion of a norovirus nucleic acid sequence.

The disclosure also encompasses kits for detecting the presence of norovirus nucleic acids in a test sample. The kit, for example, includes a labeled compound or agent capable of detecting a nucleic acid molecule in a test sample and, in certain embodiments of the present disclosure, for determining the quantity of norovirus in the sample.

For oligonucleotide-based kits, the kit includes, for example: (1) an oligonucleotide, e.g., a detectably labeled oligonucleotide, which hybridizes to a nucleic acid sequence of norovirus and/or (2) one or a pair of primers (one forward and one reverse) useful for amplifying a nucleic acid molecule containing at least a portion the norovirus sequence. The kit can also include, for example, a buffering agent, a preservative, or a protein stabilizing agent. The kit can also include components necessary for detecting the detectable agent (e.g., an enzyme or a substrate). The kit can also contain a control sample, a control organism, or a series of control samples or organisms that are assayed and compared to the test sample contained. Each component of the kit is usually enclosed within an individual container and all of the various containers are optionally enclosed within a single package along with instructions for use.

The processes are amenable to use for diagnosis of norovirus infection or simple detection of the presence or absence of norovirus in a subject, such as humans, and any other organism capable of infection or transfection by or with norovirus.

To increase confidence and to serve as an internal or external control, a purified solution containing norovirus is optionally used as a test sample, control sample, calibration sample, or other sample. Optionally, by amplification of a single sample with known quantities of norovirus or of a set of samples representing a titration of norovirus, the level of norovirus in the unknown sample is determined, optionally as a control. Optionally, the purified and quantified norovirus solution is analyzed in parallel with the unknown sample to reduce inter assay error or to serve as a standard curve for quantitation of unknown norovirus in the test sample. Using purified and quantified norovirus solution provides for a similar complete genetic base RNA strand for reverse transcription and subsequent amplification.

In some embodiments of the present disclosure, a subgenomic norovirus fragment is cloned into a plasmid and RNA run-off transcripts are then used for amplification, purification, and use as a quantitative comparator or nucleic acid calibrator. In a non-limiting example, the RNA sequence or portion thereof of norovirus is optionally amplified from a positive test sample. It is appreciated that other sequences are similarly suitable for use as a quantitative control. The known concentration of the RNA fragment is used to create a standard curve for quantitative determinations and to access amplification efficiency.

Also provided is a kit for detecting or diagnosing norovirus in a sample that contains reagents for the amplification, or direct detection of norovirus or portions thereof in a sample. An exemplary kit optionally includes a forward and reverse primer pair, and, optionally, a probe. In some embodiments of the present disclosure, the forward and reverse primers have the oligonucleotide sequence SEQ ID NOs: 1 and 2, SEQ ID NOs: 4 and 5, SEQ ID NOs: 10 and 11, and a probe of the sequence SEQ ID NO: 3, SEQ ID NO: 6, or SEQ ID NO: 12. A diagnostic kit optionally contains primers and probes that are the complements of SEQ ID NOs: 1-6 or 10-12, or that hybridize with oligonucleotides SEQ ID NOs: 1-6 or 10-12. A diagnostic kit optionally includes control nucleic acid and reagents for reverse transcription, amplification or detection of control nucleic acid. An exemplary kit optionally includes a forward and reverse primer pair, and a probe for amplification and detection of a control nucleic acid. In some embodiments of the present disclosure, the forward and reverse primers have the oligonucleotide sequence SEQ ID NOs: 7 and 8, and a probe of the sequence SEQ ID NO: 9. A diagnostic kit optionally contains primers and probes that are the complements of SEQ ID NOs: 7-9 or that hybridize with oligonucleotides SEQ ID NOs: 7-9. It is further appreciated that a diagnostic kit optionally includes ancillary reagents such as buffers, solvents, thermostable polymerases, nucleotides, and other reagents necessary and recognized in the art for amplification and detection of norovirus in a sample.

A kit for detection of norovirus infection in a subject optionally contains reagents for PCR based detection of genetic sequences of norovirus strains belonging to GI and/or GII. The components of the kits are any of the reagents described above or other necessary and non-necessary reagents known in the art for solubilization, detection, washing, storage, or other need for in a diagnostic assay kit.

Various aspects of the present disclosure are illustrated by the following non-limiting examples. The examples are for illustrative purposes and are not a limitation on any practice of the present disclosure. It will be understood that variations and modifications can be made without departing from the spirit and scope of the disclosure. While the examples are generally directed to samples derived from a human, a person having ordinary skill in the art recognizes that similar techniques and other techniques known in the art readily translate the examples to other organisms. Reagents illustrated herein are commonly cross reactive between mammalian species or alternative reagents with similar properties are commercially available, and a person of ordinary skill in the art readily understands where such reagents may be obtained.

### Example 1: RT-qPCR Assay Design

Primers for norovirus GI and GII specific amplification are designed based on norovirus sequences. Primers and probes are analyzed for homology to other known sequences using the Basic Local Alignment Search Tool (BLAST). Altschul SF, et al., J Mol Biol, 1990; 215: 403-410. BLAST results show that the primers of SEQ ID NOs: 1, 2, 4, and 5 have no homology that was over 78% nucleotide identity with any gene in the target norovirus.

To determine if the primers are capable of amplifying norovirus, primers are tested for optimal concentration in triplicate or quadruplicate by RT-PCR in combinations of final concentrations of 50, 100, 200, 400, 600, and 900 nM; the probe is tested in triplicate at final concentrations of 50, 100, 200, and 400 nM.

RT-qPCR is performed as follows: An ABI 7500 (Applied Biosystems) and AgPath-ID™ One-Step RT-PCR Kit (Applied Biosystems) are used to optimize primer concentrations. Cycle parameters are reverse transcription for 10 min at 45°C and denaturation for 10 min at 95°C, followed by amplification using 45 cycles of 15 sec at 95°C and 1 min at 60°C. For following studies, master mixes contain reagents as in Table 3:

**Table 3:**

| Component | Volume per reaction (µl) | Final concentration |
|---|---|---|
| 2X RT-PCR buffer* | 12.50 | 1X |
| Nuclease-free water* | 1.08 | n/a |
| Detection Enhancer* | 1.67 | n/a |
| Cog1F (10 µM) | 1.0 | 400 nM |
| Cog1R (10 µM) | 1.0 | 400 nM |
| Ring 1E (10 µM) | 0.5 | 200 nM |
| Cog2F (10 µM) | 1.0 | 400 nM |
| Cog2R (10 µM) | 1.0 | 400 nM |
| Ring 2(10 µM) | 0.5 | 200 nM |
| MS2.F (10 µM) | 0.25 | 100 nM |
| MS2.R (10 µM) | 0.25 | 100 nM |
| MS2.P (10 µM) | 0.25 | 100 nM |
| 25X RT-PCR enzyme* | 1.00 | 1x |
| Master Mix volume | 22 | |

The primers of SEQ ID NOs: 1(Cog1F), 2 (Cog1R), 4 (Cog2F), and 5 (Cog2R), along with the labeled probes of SEQ ID NO: 3 (RinglE), and 6 (Ring 2), successfully amplify and detect norovirus (FIG. 1). The control MS2 primers of SEQ ID NO: 7 (MS2.F) and 8 (MS2.R) as well as the MS2 probe of SEQ ID NO: 9 (MS2.P) successfully amplify MS2 added to the system at known concentration.

The reactions or Table 3 are repeated by replacing Cog1F with SEQ ID NO: 11 (Cog1NF), Cog1R with SEQ ID NO: 11 (Cog1NR), and replacing or supplementing the RinglE probe with SEQ ID NO: 12 (NV1LCpr). Similar highly specific and robust amplification of GI and GII are identified.

The reactions of Table 3 are repeated by supplementing Cog1F with SEQ ID NO: 11 (Cog1NF), Cog1R with SEQ ID NO: 11 (Cog1NR), and RinglE probe with SEQ ID NO: 12 (NV1LCpr). Similar highly specific and robust amplification of GI and GII are identified.

### Example 2: Assay for presence of norovirus in biological samples from clinical sources.

The ability of each of the norovirus assays of Example 1 to detect norovirus is assessed using extracted RNA from fecal specimens.

For the clinical samples, viral RNA is extracted from clarified 10% fecal suspensions in phosphate-buffered saline with the MagMax-96 Viral RNA Isolation Kit (Ambion, Foster City, CA, USA) on an automated KingFisher magnetic particle processor (Thermo Fisher Scientific, Pittsburgh, PA, USA) according to the manufacturer's instructions and eluted into 100 µL of elution buffer (10 mmol/L Tris pH 8.0 and 1 mmol/L EDTA). Extracted RNA is stored at -80°C until further use or stored on ice and assayed within 30 minutes of isolation. The RT-qPCR assay of Example 1 is used to examine each of the samples for the presence or absence of norovirus.

### Example 3: Detection of norovirus by PCR/LC/MS

The samples of Example 2 are each rescreened using PCR amplification with parameters similar to the RT-qPCR assay of Example 1. The reaction products are subjected to analyses by electrospray ionization mass spectrometry substantially as described by Naito, Y, et al., Rapid Communications in Mass Spectrometry, 1995; 9:1484-1486; or Wunschel DS, et al., Rapid Common Mass Spectrom. 1996; 10(1):29-35. Each of the reaction products from the PCR reactions are successfully and rapidly detected.

### Example 4: Detection of norovirus by PCR/gel electrophoresis

The samples of Example 2 are each rescreened using PCR amplification with parameters similar to the RT-qPCR assay of Example 1. The amplified reaction products are separated by gel electrophoresis and detected by fluorescent imaging. Each of the isolates show detectable amplified DNA.

Methods involving conventional biological techniques are described herein. Such techniques are generally known in the art and are described in detail in methodology treatises such as Molecular Cloning: A Laboratory Manual, 3rd ed., vol. 1-3, ed. Sambrook et al., Cold Spring Harbor Laboratory Press, Cold Spring Harbor, N.Y., 2001; Current Protocols in Molecular Biology, ed. Ausubel et al., Greene Publishing and Wiley-Interscience, New York, 1992 (with periodic updates); and Short Protocols in Molecular Biology, ed. Ausubel et al., 52 ed., Wiley-Interscience, New York, 2002. Immunological methods (e.g., preparation of antigen-specific antibodies, immunoprecipitation, and immunoblotting) are described, e.g., in Current Protocols in Immunology, ed. Coligan et al., John Wiley & Sons, New York, 1991; and Methods of Immunological Analysis, ed. Masseyeff et al., John Wiley & Sons, New York, 1992.

Additional protocols such as PCR Protocols can be found in A Guide to Methods and Applications Academic Press, NY.

It is appreciated that all reagents are obtainable by sources known in the art unless otherwise specified. Methods of nucleotide amplification, cell transfection, and purification are similarly within the level of skill in the art.

### REFERENCE LIST

1. Scallan E, Hoekstra RM, Angulo FJ, Tauxe RV, Widdowson MA, Roy SL, Foodborne illness acquired in the United States-major pathogens. Emerg Infect Dis. 2011;17:7-15.
2. Glass RI, Parashar UD, Estes MK. Norovirus gastroenteritis. N Engl J Med. 2009;361:1776-85.
3. Centers for Disease Control and Prevention. Surveillance for foodborne disease outbreaks-United States, 2006. MMWR Morb Mortal Wkly Rep. 2009;58:609-15.
4. Wilhelm CM, Hanna SL, Welch CA, Shahid H, Minnich LL, Daly SB, Viral gastroenteritis in Charleston, West Virginia, in 2007: from birth to 99 years of age. Infect Control Hosp Epidemiol. 2010;31:816-21.
5. Roddie C, Paul JP, Benjamin R, Gallimore CI, Xerry J, Gray JJ, Allogeneic hematopoietic stem cell transplantation and norovirus gastroenteritis: a previously unrecognized cause of morbidity. Clin Infect Dis. 2009;49:1061-8.
6. Haustein T, Harris JP, Pebody R, Lopman BA. Hospital admissions due to norovirus in adult and elderly patients in England. Clin Infect Dis. 2009;49:1890-2.
7. Lopman BA, Reacher MH, Vipond IB, Sarangi J, Brown DW. Clinical manifestation of norovirus gastroenteritis in health care settings. Clin Infect Dis. 2004;39:318-24.
8. Rosenthal NA, Lee LE, Vermeulen BA, Hedberg K, Keene WE, Widdowson MA, Epidemiological and genetic characteristics of norovirus outbreaks in long-term care facilities, 2003-2006. Epidemiol Infect. 2011;139:286-94.
9. Johnston CP, Qiu H, Ticehurst JR, Dickson C, Rosenbaum P, Lawson P, Outbreak management and implications of a nosocomial norovirus outbreak. Clin Infect Dis. 2007;45:534-40.
10. Ethelberg S, Lisby M, Bottiger B, Schultz AC, Villif A, Jensen T, Outbreaks of gastroenteritis linked to lettuce, Denmark, January 2010. Euro Surveill. 2010;15:pii:19484.
11. Lopman BA, Hall AJ, Curns A, Parashar UD. Increasing rates of gastroenteritis hospital discharges in US adults and the contribution of norovirus, 1996 to 2007. Clin Infect Dis. 2011;52:466-74.
12. Mounts AW, Ando T, Koopmans M, Bresee JS, Noel J, Glass RI. Cold weather seasonality of gastroenteritis associated with Norwalk-like viruses. J Infect Dis. 2000;181(Supp1 2):S284-7.
13. Fankhauser RL, Monroe SS, Noel JS, Humphrey CD, Bresee JS, Parashar UD, Epidemiologic and molecular trends of "Norwalk-like viruses" associated with outbreaks of gastroenteritis in the United States. J Infect Dis. 2002;186:1-7.
14. Zheng DP, Widdowson MA, Glass RI, Vinjé J. Molecular epidemiology of genogroup II-genotype 4 noroviruses in the United States between 1994 and 2006. J Clin Microbiol. 2010;48:168-77.
15. Siebenga JJ, Vennema H, Zheng DP, Vinjé J, Lee BE, Pang XL, Norovirus illness is a global problem: emergence and spread of norovirus GII.4 variants, 2001-2007. J Infect Dis. 2009;200:802-12.
16. Zheng DP, Ando T, Fankhauser RL, Beard RS, Glass RI, Monroe SS. Norovirus classification and proposed strain nomenclature. Virology. 2006;346:312-23.
17. Svraka S, Vennema H, van der Veer B, Hedlund KO, Thorhagen M, Siebenga J, Epidemiology and genotype analysis of emerging sapovirus-associated infections across. Eur J Clin Microbiol. 2010;48:2191-8.
18. Finkbeiner SR, Li Y, Ruone S, Conrardy C, Gregoricus N, Toney D, Identification of a novel astrovirus (astrovirus VA1) associated with an outbreak of acute gastroenteritis. J Virol. 2009;83:10836-9.
19. Cunliffe NA, Booth JA, Elliot C, Lowe SJ, Sopwith W, Kitchin N, Healthcare-associated viral gastroenteritis among children in a large pediatric hospital, United Kingdom. Emerg Infect Dis. 2010;16:55-62.
20. Donaldson EF, Lindesmith LC, Lobue AD, Baric RS. Viral shape-shifting: norovirus evasion of the human immune system. Nat Rev Microbiol. 2010;8:231-41.
21. Centers for Disease Control and Prevention. Norovirus activity-United States, 2006-2007. MMWR Morb Mortal Wkly Rep. 2007;56:842-6.
22. Lindesmith LC, Donaldson EF, Baric RS. Norovirus GII.4 strain antigenic variation. J Virol. 2011;85:231-42.
23. Lindesmith L, Moe C, Marionneau S, Ruvoen N, Jiang X, Lindblad L, Human susceptibility and resistance to Norwalk virus infection. Nat Med. 2003;9:548-53.
24. Fankhauser RL, Noel JS, Monroe SS, Ando T, Glass RI. Molecular epidemiology of "Norwalk-like viruses" in outbreaks of gastroenteritis in the United States. J Infect Dis. 1998;178:1571-8.
25. Blanton LH, Adams SM, Beard RS, Wei G, Bulens SN, Widdowson MA, Molecular and epidemiologic trends of caliciviruses associated with outbreaks of acute gastroenteritis in the United States, 2000-2004. J Infect Dis. 2006;193:413-21.
26. Vinjé J, Hamidjaja RA, Sobsey MD. Development and application of a capsid VP1 (region D) based reverse transcription PCR assay for genotyping of genogroup I and II noroviruses. J Virol Methods. 2004;116:109-17.
27. Trujillo AA, McCaustland KA, Zheng DP, Hadley LA, Vaughn G, Adams SM, Use of TaqMan real-time reverse transcription-PCR for rapid detection, quantification, and typing of norovirus. J Clin Microbiol. 2006;44:1405-12.
28. Hill VR, Mull B, Jothikumar N, Ferdinand K, Vinjé J. Norovirus detection in ground water using ultrafiltration and real-time RT-PCR. Food Environ Virol. 2011. In press. http://www.springerlink.com/content/1867-0334/2/4/
29. Ando T, Monroe SS, Noel JS, Glass RI. A one-tube method of reverse transcription-PCR to efficiently amplify a 3-kilobase region from the RNA polymerase gene to the poly(A) tail of small round-structured viruses (Norwalk-like viruses). J Clin Microbiol. 1997;35:570-7.
30. Tamura K, Dudley J, Nei M, Kumar S. MEGA4: Molecular Evolutionary Genetics Analysis (MEGA) software version 4.0. Mol Biol Evol. 2007;24:1596-9.
31. Guindon S, Gascuel O. A simple, fast, and accurate algorithm to estimate large phylogenies by maximum likelihood. Syst Biol. 2003;52:696-704.
32. Posada D. jModelTest: phylogenetic model averaging. Mol Biol Evol. 2008;25:1253-6.
33. Anisimova M, Gascuel O. Approximate likelihood-ratio test for branches: a fast, accurate, and powerful alternative. Syst Biol. 2006;55:539-52.
34. US Census Bureau. Annual estimates of the resident population for the United States, regions, states, and Puerto Rico: April 1, 2000 to July 1, 2009 [cited 2010 Jun 29]. http://www.census.gov/popest/states/NST-ann-est.html
35. Vega E, Vinjé J. Novel GII.12 norovirus strain, United States, 2009-2010. Emerg Infect Dis. 2011;17:1516-8.
36. Yang Y, Xia M, Tan M, Huang P, Zhong W, Pang XL, Genetic and phenotypic characterization of GII-4 noroviruses that circulated during 1987 to 2008. J Virol. 2010;84:9595-607.
37. Swaminathan B, Barrett TJ, Hunter SB, Tauxe RV. CDC PulseNet Task Force. PulseNet: the molecular subtyping network for foodborne bacterial disease surveillance, United States. Emerg Infect Dis. 2001;7:382-9.
38. Xerry J, Gallimore CI, Iturriza-Gómara M, Allen DJ, Gray JJ. Transmission events within outbreaks of gastroenteritis determined through analysis of nucleotide sequences of the P2 domain of genogroup II noroviruses. J Clin Microbiol. 2008;46:947-53.
39. Koopmans M, Vennema H, Heersma H, van Strien E, van Duynhoven Y, Brown D, Early identification of common-source foodborne virus outbreaks in Europe. Emerg Infect Dis. 2003;9:1136-42.
40. Vega E, Barclay L, Gregoricus N, Williams K, Lee D, Vinjé J. Novel surveillance network for norovirus gastroenteritis outbreaks, United States. Emerg Infect Dis [serial on the Internet]. 2011 Aug [November 9, 2011]. http://dx.doi.org/10.3201/eid1708.101837
41. Schultz AC, Vega E, Dalsgaard A, Christensen LS, N⌀rrung B, Hoorfar J, Vinjé J, Development and evaluation of novel one-step TaqMan realtime RT-PCR assays for the detection and direct genotyping of genogroup I and II noroviruses. J Clin Virol. 2011 Mar;50(3):230-4.
42. Svraka S, Duizer E, Vennema H, de Bruin E, van der Veer B, Dorresteijn B,Koopmans M. Etiological role of viruses in outbreaks of acute gastroenteritis in The Netherlands from 1994 through 2005. J Clin Microbiol. 2007 May;45(5):1389-94.

Patents and publications mentioned in the specification are indicative of the levels of those skilled in the art to which the disclosure pertains.

### SEQUENCE LISTING

<110> The Government of the United States of America as represented by the Secretary of the Department of Health and Human Services, Centers for Disease Control and Prevention
<120> SELECTIVE DETECTION OF NOROVIRUS
<130> CDC-18152/38
<150> US 61/560,077
   <151> 2011-11-15
<160> 12
<170> PatentIn version 3.5
<210> 1
   <211> 20
   <212> DNA
   <213> Artificial Sequence
<220>
   <223> norovirus
<220>
   <221> modified_base
   <222> (12)..(12)
   <223> i
<400> 1
   cgytggatgc gittycatga 20
<210> 2
   <211> 22
   <212> DNA
   <213> Artificial Sequence
<220>
   <223> norovirus
<400> 2
   cttagacgcc atcatcatty ac 22
<210> 3
   <211> 15
   <212> DNA
   <213> Artificial Sequence
<220>
   <223> norovirus
<400> 3
   tggacaggrg aycgc 15
<210> 4
   <211> 26
   <212> DNA
   <213> Artificial Sequence
<220>
   <223> norovirus
<220>
   <221> misc_feature
   <222> (9) .. (9)
   <223> n is a, c, g, t or u
<400> 4
   cargarbcna tgttyagrtg gatgag 26
<210> 5
   <211> 21
   <212> DNA
   <213> Artificial Sequence
<220>
   <223> norovirus
<400> 5
   tcgacgccat cttcattcac a 21
<210> 6
   <211> 20
   <212> DNA
   <213> Artificial Sequence
<220>
   <223> norovirus
<400> 6
   tgggagggcg atcgcaatct 20
<210> 7
   <211> 26
   <212> DNA
   <213> Artificial Sequence
<220>
   <223> norovirus
<400> 7
   tggcactacc cctctccgta ttcacg 26
<210> 8
   <211> 22
   <212> DNA
   <213> Artificial Sequence
<220>
   <223> norovirus
<400> 8
   gtacgggcga ccccacgatg ac 22
<210> 9
   <211> 29
   <212> DNA
   <213> Artificial Sequence
<220>
   <223> norovirus
<400> 9
   cacatcgata gatcaaggtg cctacaagc 29
<210> 10
   <211> 18
   <212> DNA
   <213> Artificial Sequence
<220>
   <223> norovirus
<400> 10
   ccatgttccg ttggatgc 18
<210> 11
   <211> 20
   <212> DNA
   <213> Artificial Sequence
<220>
   <223> norovirus
<400> 11
   tccttagacg ccatcatcat 20
<210> 12
   <211> 19
   <212> DNA
   <213> Artificial Sequence
<220>
   <223> norovirus
<400> 12
   ggacaggaga ycgcratct 19

## Claims

1. An *in vitro* process of detecting norovirus in a sample comprising:
producing an amplification product by amplifying a norovirus nucleotide sequence using a forward primer that hybridizes to a norovirus nucleotide sequence, and a reverse primer that hybridizes to a region within the norovirus nucleotide sequence, under conditions suitable for a polymerase chain reaction; and
detecting said amplification product to detect the norovirus in the sample using a first probe consisting of a fluorescent label, a quencher and a nucleic acid sequence consisting of SEQ ID NO: 3.

2. The process of claim 1 wherein said forward primer comprises the sequence of SEQ ID NO: 1, SEQ ID NO: 4, SEQ ID NO: 10, or combinations thereof.

3. The process of claims 1 or 2 wherein said reverse primer comprises the sequence of SEQ ID NO: 2, SEQ ID NO: 5, SEQ ID NO: 11, or combinations thereof.

4. The process of claims 1 or 2 wherein said detecting is by using a second probe comprising the sequence of SEQ ID NO: 3, SEQ ID NO: 6, SEQ ID NO: 12, or combinations thereof.

5. The process of claims 1 or 2 wherein hybridizing said first probe is under conditions suitable for a polymerase chain reaction; and further
detecting a first detection signal from said probe hybridized to said amplification product.

6. The process of claim 1 further comprising
adding a quantity of control organism to said sample,
producing a control amplification product by amplifying a control nucleotide sequence using a control forward primer that hybridizes to a control nucleotide sequence, and a control reverse primer that hybridizes to a region within the control nucleotide sequence, under conditions suitable for a polymerase chain reaction; and
detecting said control amplification product to detect the control organism in the sample.

7. The process of claim 6 wherein said control organism is an RNA virus.

8. The process of claim 6 wherein said control organism is RNA coliphage MS2.

9. A kit for detecting norovirus infection in a subject comprising:
a forward primer comprising sequence of SEQ ID NO: 1, SEQ ID NO: 4, SEQ ID NO: 7, SEQ ID NO: 10, or a combination of said forward primers;
a reverse primer comprising the sequence of SEQ ID NO: 2, SEQ ID NO: 5, SEQ ID NO: 8, SEQ ID NO: 11, or a combination said reverse primers; and
a probe_consisting of a fluorescent label a quencher, and a nucleotide sequence consisting of SEQ ID NO: 3.

10. An isolated oligonucleotide consisting of a fluorescent label, a quencher, and a nucleotide sequence consisting of SEQ ID NO: 3.

11. A process of detecting the presence or absence of norovirus in a sample comprising:
obtaining a sample;
contacting said sample with a first forward primer that hybridizes to a sequence of a genogroup I norovirus and a first reverse primer that hybridizes to a sequence of a genogroup I norovirus, under conditions suitable for a polymerase chain reaction;
contacting said sample with a second forward primer that hybridizes to a sequence of a genogroup II norovirus and a second reverse primer that hybridizes to a sequence of a genogroup II norovirus, under conditions suitable for a polymerase chain reaction;
contacting said sample with a third forward primer that hybridizes to a sequence of a coliphage MS2 and a third reverse primer that hybridizes to a sequence of a coliphage MS2 under conditions suitable for a polymerase chain reaction; and
detecting the presence or absence of norovirus in said sample by detecting the presence or absence of an amplification product produced from said steps of contacting, said step of detecting using a first probe consisting of a fluorescent label, a quencher, and a nucleotide sequence consisting of SEQ ID NO: 3.

12. The process of claim 11 further comprising adding a quantity of coliphage MS2 to said sample prior to said steps of contacting.

13. The process of claim 11 wherein said detecting is further by using a probe comprising the sequence of SEQ ID NO: 6, SEQ ID NO: 9, or SEQ ID NO: 12, said probe producing a detection signal when hybridized to an amplification product.

14. The process of claims 1 or 11, or the composition of claims 9 or 10 wherein the quencher is a dihydrocyclopyrroloindole tripeptide minor groove binder.

15. A process of detecting the presence or absence of norovirus in a sample comprising:
obtaining a sample;
contacting said sample with a first forward primer comprising SEQ ID NO: 1 and a first reverse primer comprising SEQ ID NO: 2;
contacting said sample with a second forward primer that comprises SEQ ID NO: 4 and a second reverse primer that comprises SEQ ID NO: 5;
contacting said sample with a third forward primer that comprises SEQ ID NO: 7 and a third reverse primer that comprises SEQ ID NO: 8; and
detecting the presence or absence of norovirus in said sample by detecting the presence or absence of an amplification product produced from said steps of contacting, said step of detecting using a first probe consisting of a fluorescent label, a quencher, and a nucleotide sequence consisting of SEQ ID NO: 3, a second probe comprising a fluorescent label, a quencher and a nucleotide sequence of SEQ ID NO: 6, and a third probe comprising a fluorescent label, a quencher and a nucleotide sequence of SEQ ID NO: 9.

## Patentansprüche

1. In-vitro-Verfahren des Erkennens eines Norovirus in einer Probe, Folgendes umfassend:
Herstellen eines Amplifikationsprodukts durch Amplifizieren einer Norovirus-Nukleotidsequenz unter Verwendung eines Vorwärtsprimers, der sich an eine Norovirus-Nukleotidsequenz hybridisiert, und eines Rückwärtsprimers, der sich an eine Region innerhalb der Norovirus-Nukleotidsequenz hybridisiert, unter Bedingungen, die für eine Polymerase-Kettenreaktion geeignet sind; und
Erkennen des Amplifikationsprodukts, um den Norovirus in der Probe zu erkennen, unter Verwendung einer ersten Sonde, bestehend aus einer Fluoreszenzmarkierung, einem Quencher und einer Nukleinsäuresequenz, die aus SEQ ID NO: 3 besteht.

2. Verfahren nach Anspruch 1, wobei der Vorwärtsprimer die Sequenz SEQ ID NO: 1, SEQ ID NO: 4, SEQ ID NO: 10 oder Kombinationen davon umfasst.

3. Verfahren nach Ansprüche 1 oder 2, wobei der Rückwärtsprimer die Sequenz SEQ ID NO: 2, SEQ ID NO: 5, SEQ ID NO: 11 oder Kombinationen davon umfasst.

4. Verfahren nach Ansprüche 1 oder 2, wobei das Erkennen unter Verwendung einer zweiten Sonde, die die Sequenz SEQ ID NO: 3, SEQ ID NO: 6, SEQ ID NO: 12 oder Kombinationen davon umfasst, stattfindet.

5. Verfahren nach Ansprüche 1 oder 2, wobei das Hybridisieren der ersten Sonde unter Bedingungen stattfindet, die für eine Polymerase-Kettenreaktion geeignet sind; und ferner
Erkennen eines ersten Erkennungssignals von der Sonde, die an das Amplifikationsprodukt hybridisiert ist.

6. Verfahren nach Anspruch 1, ferner umfassend:
Hinzufügen einer Menge eines Kontrollorganismus zu der Probe,
Herstellen eines Kontrollamplifikationsprodukts durch Amplifizieren einer Kontroll-Nukleotidsequenz unter Verwendung eines Kontroll-Vorwärtsprimers, der sich an eine Kontroll-Nukleotidsequenz hybridisiert, und eines Kontroll-Rückwärtsprimers, der sich an eine Region innerhalb der Kontroll-Nukleotidsequenz hybridisiert, unter Bedingungen, die für eine Polymerase-Kettenreaktion geeignet sind; und
Erkennen des Kontrollamplifikationsprodukts, um den Kontrollorganismus in der Probe zu erkennen.

7. Verfahren nach Anspruch 6, wobei der Kontrollorganismus ein RNA-Virus ist.

8. Verfahren nach Anspruch 6, wobei der Kontrollorganismus RNA-Coliphage MS2 ist.

9. Satz zum Erkennen einer Norovirus-Infektion in einem Subjekt, Folgendes umfassend:
einen Vorwärtsprimer, der die Sequenz SEQ ID NO: 1, SEQ ID NO: 4, SEQ ID NO: 7, SEQ ID NO: 10 oder eine Kombination der Vorwärtsprimer umfasst;
einen Rückwärtsprimer, der die Sequenz SEQ ID NO: 2, SEQ ID NO: 5, SEQ ID NO: 8, SEQ ID NO: 11 oder eine Kombination der Rückwärtsprimer umfasst; und
eine Sonde, bestehend aus einer Fluoreszenzmarkierung, einem Quencher und einer Nukleotidsequenz, die aus SEQ ID NO: 3 besteht.

10. Isoliertes Oligonukleotid, bestehend aus einer Fluoreszenzmarkierung, einem Quencher und einer Nukleotidsequenz, die aus SEQ ID NO: 3 besteht.

11. Verfahren zum Erkennen des Vorhandenseins oder Nichtvorhandenseins eines Norovirus in einer Probe, Folgendes umfassend:
Entnehmen einer Probe;
Inkontaktbringen der Probe mit einem ersten Vorwärtsprimer, der an eine Sequenz eines Norovirus Genogruppe I hybridisiert, und einem ersten Rückwärtsprimer, der an eine Sequenz eines Norovirus Genogruppe I hybridisiert, unter Bedingungen, die für eine Polymerase-Kettenreaktion geeignet sind;
Inkontaktbringen der Probe mit einem zweiten Vorwärtsprimer, der an eine Sequenz eines Norovirus Genogruppe II hybridisiert, und einem zweiten Rückwärtsprimer, der an eine Sequenz eines Norovirus Genogruppe II hybridisiert, unter Bedingungen, die für eine Polymerase-Kettenreaktion geeignet sind;
Inkontaktbringen der Probe mit einem dritten Vorwärtsprimer, der an eine Sequenz eines Coliphage MS2 hybridisiert, und einem dritten Rückwärtsprimer, der an eine Sequenz eines Coliphage MS2 hybridisiert, unter Bedingungen, die für eine Polymerase-Kettenreaktion geeignet sind; und
Erkennen des Vorhandenseins oder Nichtvorhandenseins eines Norovirus in der Probe durch Erkennen des Vorhandenseins oder Nichtvorhandenseins eines Amplifikationsprodukts, das aus den Schritten des Inkontaktbringens hergestellt wurde, wobei der Schritt des Erkennens unter Verwendung einer ersten Sonde, bestehend aus einer Fluoreszenzmarkierung, einem Quencher und einer Nukleotidsequenz, die aus SEQ ID NO: 3 besteht, stattfindet.

12. Verfahren nach Anspruch 11, ferner umfassend das Hinzufügen einer Menge Coliphage MS2 zu der Probe vor den Schritten des Inkontaktbringens.

13. Verfahren nach Anspruch 11, wobei das Erkennen ferner unter Verwendung einer Sonde stattfindet, die die Sequenz SEQ ID NO: 6, SEQ ID NO: 9 oder SEQ ID NO: 12 umfasst, wobei die Sonde ein Erkennungssignal erzeugt, wenn sie an ein Amplifikationsprodukt hybridisiert ist.

14. Verfahren nach Ansprüche 1 oder 11, oder die Zusammensetzung von Ansprüchen 9 oder 10, wobei der Quencher ein Dihydrocyclopyrroloindol-Tripeptid Minor-Groove-Binder ist.

15. Verfahren zum Erkennen des Vorhandenseins oder Nichtvorhandenseins eines Norovirus in einer Probe, Folgendes umfassend:
Entnehmen einer Probe;
Inkontaktbringen der Probe mit einem ersten Vorwärtsprimer, umfassend SEQ ID NO: 1, und einem ersten Rückwärtsprimer, umfassend SEQ ID NO: 2;
Inkontaktbringen der Probe mit einem zweiten Vorwärtsprimer, umfassend SEQ ID NO: 4, und einem zweiten Rückwärtsprimer, umfassend SEQ ID NO: 5;
Inkontaktbringen der Probe mit einem dritten Vorwärtsprimer, umfassend SEQ ID NO: 7, und einem dritten Rückwärtsprimer, umfassend SEQ ID NO: 8; und
Erkennen des Vorhandenseins oder Nichtvorhandenseins eines Norovirus in der Probe durch Erkennen des Vorhandenseins oder Nichtvorhandenseins eines Amplifikationsprodukts, das aus den Schritten des Inkontaktbringens hergestellt wurde, wobei der Schritt des Erkennens unter Verwendung einer ersten Sonde, bestehend aus einer Fluoreszenzmarkierung, einem Quencher und einer Nukleotidsequenz, die aus SEQ ID NO: 3 besteht, einer zweiten Sonde, bestehend aus einer Fluoreszenzmarkierung, einem Quencher und einer Nukleotidsequenz, die aus SEQ ID NO: 6 besteht, und einer dritten Sonde, bestehend aus einer Fluoreszenzmarkierung, einem Quencher und einer Nukleotidsequenz, die aus SEQ ID NO: 9 besteht, stattfindet.

## Revendications

1. Procédé *in vitro* de détection de norovirus dans un échantillon comprenant :
la production d'un produit d'amplification par amplification d'une séquence nucléotidique de norovirus en utilisant une amorce directe qui s'hybride à une séquence nucléotidique de norovirus, et une amorce inverse qui s'hybride à une région au sein de la séquence nucléotidique de norovirus, dans des conditions appropriées pour une réaction en chaîne par polymérase ; et
la détection dudit produit d'amplification pour détecter le norovirus dans l'échantillon en utilisant une première sonde constituée d'un marqueur fluorescent, d'un extincteur et d'une séquence d'acide nucléique constituée de SEQ ID NO : 3.

2. Procédé selon la revendication 1 dans lequel ladite amorce directe comprend la séquence de SEQ ID NO : 1, SEQ ID NO : 4, SEQ ID NO : 10, ou leurs combinaisons.

3. Procédé selon les revendications 1 ou 2 dans lequel ladite amorce inverse comprend la séquence de SEQ ID NO : 2, SEQ ID NO : 5, SEQ ID NO : 11, ou leurs combinaisons.

4. Procédé selon les revendications 1 ou 2 dans lequel ladite détection est réalisée par l'utilisation d'une seconde sonde comprenant la séquence de SEQ ID NO : 3, SEQ ID NO : 6, SEQ ID NO : 12, ou leurs combinaisons.

5. Procédé selon les revendications 1 ou 2 dans lequel l'hybridation de ladite première sonde est réalisée dans des conditions appropriées pour une réaction en chaîne par polymérase ; et en outre
la détection d'un premier signal de détection provenant de ladite sonde hybridée au dit produit d'amplification.

6. Procédé selon la revendication 1 comprenant en outre
l'ajout d'une quantité d'organisme témoin audit échantillon,
la production d'un produit d'amplification témoin par amplification d'une séquence nucléotidique témoin en utilisant une amorce directe témoin qui s'hybride à une séquence nucléotidique témoin, et une amorce inverse témoin qui s'hybride à une région au sein de la séquence nucléotidique témoin, dans des conditions appropriées pour une réaction en chaîne par polymérase ; et
la détection dudit produit d'amplification témoin pour détecter l'organisme témoin dans l'échantillon.

7. Procédé selon la revendication 6 dans lequel ledit organisme témoin est un virus à ARN.

8. Procédé selon la revendication 6 dans lequel ledit organisme témoin est le coliphage MS2 à ARN.

9. Kit pour la détection d'une infection à norovirus chez un sujet comprenant :
une amorce directe comprenant la séquence de SEQ ID NO : 1, SEQ ID NO : 4, SEQ ID NO : 7, SEQ ID NO : 10, ou une combinaison desdites amorces directes ;
une amorce inverse comprenant la séquence de SEQ ID NO : 2, SEQ ID NO : 5, SEQ ID NO : 8, SEQ ID NO : 11, ou une combinaison desdites amorces inverses ; et
une sonde constituée d'un marqueur fluorescent, d'un extincteur et d'une séquence nucléotidique constituée de SEQ ID NO : 3.

10. Oligonucléotide isolé constitué d'un marqueur fluorescent, d'un extincteur, et d'une séquence nucléotidique constituée de SEQ ID NO : 3.

11. Procédé de détection de la présence ou de l'absence de norovirus dans un échantillon comprenant :
l'obtention d'un échantillon ;
la mise en contact dudit échantillon avec une première amorce directe qui s'hybride à une séquence d'un norovirus du génogroupe I et une première amorce inverse qui s'hybride à une séquence d'un norovirus du génogroupe I, dans des conditions appropriées pour une réaction en chaîne par polymérase ;
la mise en contact dudit échantillon avec une deuxième amorce directe qui s'hybride à une séquence d'un norovirus du génogroupe II et une deuxième amorce inverse qui s'hybride à une séquence d'un norovirus du génogroupe II, dans des conditions appropriées pour une réaction en chaîne par polymérase ;
la mise en contact dudit échantillon avec une troisième amorce directe qui s'hybride à une séquence d'un norovirus du coliphage MS2 et une troisième amorce inverse qui s'hybride à une séquence d'un norovirus du coliphage MS2, dans des conditions appropriées pour une réaction en chaîne par polymérase ; et
la détection de la présence ou de l'absence de norovirus dans ledit échantillon par la détection de la présence ou de l'absence d'un produit d'amplification produit à partir desdites étapes de mise en contact, ladite étape de détection utilisant une première sonde constituée d'un marqueur fluorescent, d'un extincteur, et d'une séquence nucléotidique constituée de SEQ ID NO : 3.

12. Procédé selon la revendication 11 comprenant en outre l'ajout d'une quantité de coliphage MS2 audit échantillon avant lesdites étapes de mise en contact.

13. Procédé selon la revendication 11 dans lequel ladite détection est en outre réalisée en utilisant une sonde comprenant la séquence de SEQ ID NO : 6, SEQ ID NO : 9, ou SEQ ID NO : 12, ladite sonde produisant un signal de détection lorsqu'elle est hybridée à un produit d'amplification.

14. Procédé selon les revendications 1 ou 11, ou composition selon les revendications 9 ou 10 dans lequel/laquelle l'extincteur est un dihydrocyclopyrroloindole tripeptide lieur du sillon mineur.

15. Procédé de détection de la présence ou de l'absence de norovirus dans un échantillon comprenant :
l'obtention d'un échantillon ;
la mise en contact dudit échantillon avec une première amorce directe comprenant SEQ ID NO : 1 et une première amorce inverse comprenant SEQ ID NO : 2 ;
la mise en contact dudit échantillon avec une deuxième amorce directe comprenant SEQ ID NO : 4 et une deuxième amorce inverse comprenant SEQ ID NO : 5 ;
la mise en contact dudit échantillon avec une troisième amorce directe comprenant SEQ ID NO : 7 et une troisième amorce inverse comprenant SEQ ID NO : 8 ; et
la détection de la présence ou de l'absence de norovirus dans ledit échantillon par la détection de la présence ou de l'absence d'un produit d'amplification produit à partir desdites étapes de mise en contact, ladite étape de détection utilisant une première sonde constituée d'un marqueur fluorescent, d'un extincteur, et d'une séquence nucléotidique constituée de SEQ ID NO : 3, une deuxième sonde constituée d'un marqueur fluorescent, d'un extincteur, et d'une séquence nucléotidique constituée de SEQ ID NO : 6, et une troisième sonde constituée d'un marqueur fluorescent, d'un extincteur, et d'une séquence nucléotidique constituée de SEQ ID NO : 9.
